# EUROPEAN PATENT SPECIFICATION

(11) **EP 2 853 286 B1**
(45) Date of publication and mention of the grant of the patent: **18.11.2015**
(21) Application number: 13425128.9
(22) Date of filing: 25.09.2013
(51) Int. Cl.: A61N 1/06, A61N 1/32

(54) **Device for treatment of perimplantitis**
Vorrichtung zur Behandlung von Periimplantitis
Dispositif pour le traitement de perimplantitis

(43) Date of publication of application: 01.04.2015
(73) Proprietor: INQBA SRL, 60033 Chiaravalle (AN) (IT)
(72) Inventor: Tricarico, Gerardo, 60019 Senigallia (AN) (IT)
(74) Representative: Baldi, Claudio

(56) References cited:
- WO-A1-01/51122
- WO-A1-95/33416
- US-A- 4 175 565
- US-A- 5 030 236

## Description

The present patent application for industrial invention relates to a device for treatment of perimplantitis.

Implant surgery is getting more and more used to replace dental elements that are missing. Therefore, osteointegrated dental implants are used by a growing percentage of individuals.

One of the main problems that afflict said osteointegrated dental implants is the onset of perimplantitis.

Perimplantitis is caused by the accumulation of bacteria on the surface of osteointegrated implants, which causes an inflammation state known as perimplantitis.

This inflammatory process determines a progressive loss of the bone tissue that supports the osteointegrated implant until the implant is lost.

Treatments that are currently used to treat perimplantitis can be of pharmacological (non-invasive) type or of surgery (invasive) type.

A first type of surgical treatment for perimplantitis consists in cutting the inflamed gum in correspondence of the osteointegrated implant, in such manner to remove the infected portion of the gum and graft heterologous bone material.

Then, a reabsorbable membrane is inserted and stitches are given.

A second type of surgical treatment for perimplantitis provides for laser using; the objective is to eliminate the bacteria that cause perimplantitis and remove the damaged perimplantar tissue.

Such treatments are very invasive for the patient, who undergoes expensive, long and painful surgical operations that often lack efficacy over time, as shown by the data contained in the scientific literature.

US4,175,565 **discloses an electro-conductive dental implant having a cathode permanently imbedded in the jawbone, and an anode connected to a skin of the patent and to a source. A continuous current of 200 microamperes is applied intermittently for short periods, in order to increase the rate of the normal healing process.**

WO01/51122 **discloses an electrotherapy apparatus for providing two oscillating or pulsing electric alternating currents of frequencies which differ from each other by 1-250 Hz, at frequency of 1 KHz.**

The purpose of the present invention is to remedy the drawbacks of the aforementioned surgical techniques, by disclosing a device for treatment of perimplantitis that is efficacious, easy to use and able to treat perimplantitis in a not very invasive way for the patient.

While carrying out tests and experiments the inventor has surprisingly discovered that perimplantitis can be treated by delivering high-frequency alternating current on the damaged tissue, using the dental implant as current dissipator terminal.

The inventor has discovered that the best results are obtained if a constant power dissipation of about 200W is maintained on the damaged tissue.

However, the power dissipated on the damaged tissue varies considerably according to the type of tissue and severity of perimplantitis.

The inventor has realized that the variation of power dissipated on the damaged tissue strictly depends on the variation of electrical resistance of the damaged tissue.

Based on such a consideration the inventor has developed a device for treatment of perimplantitis able to detect the electrical resistance of the damaged tissue and, according to the resistance value detected, modulate the characteristics of an electrical pulse train at alternating current and high-frequency delivered through the implant to the damaged tissue.

The inventor has advantageously discovered that the duration and frequency of the pulse train could be modulated according to the extension of the damaged tissue and to the electrical affinity of the damaged tissue. Therefore, the device can optionally detect the extension and electrical affinity of the damaged tissue and accordingly modulate the characteristics of the high-frequency alternating current electrical pulse train delivered through the implant to the damaged tissue.

Additional characteristics of the invention will appear evident from the detailed description below, with reference to the attached drawings, which have an illustrative, not limitative purpose only, wherein:
Fig. 1 is a diagrammatic sectional view of an intraosseous dental implant and damaged tissue affected by perimplantitis;
Fig. 2 is a block diagram showing the device of the invention; and
Fig. 3 is a chart showing an electrical pulse train generated by the pulse generator of the device of the invention;
Fig. 4 is an axonometric view of the device of the invention, which comprises handpiece, main electrode and neutral electrode.

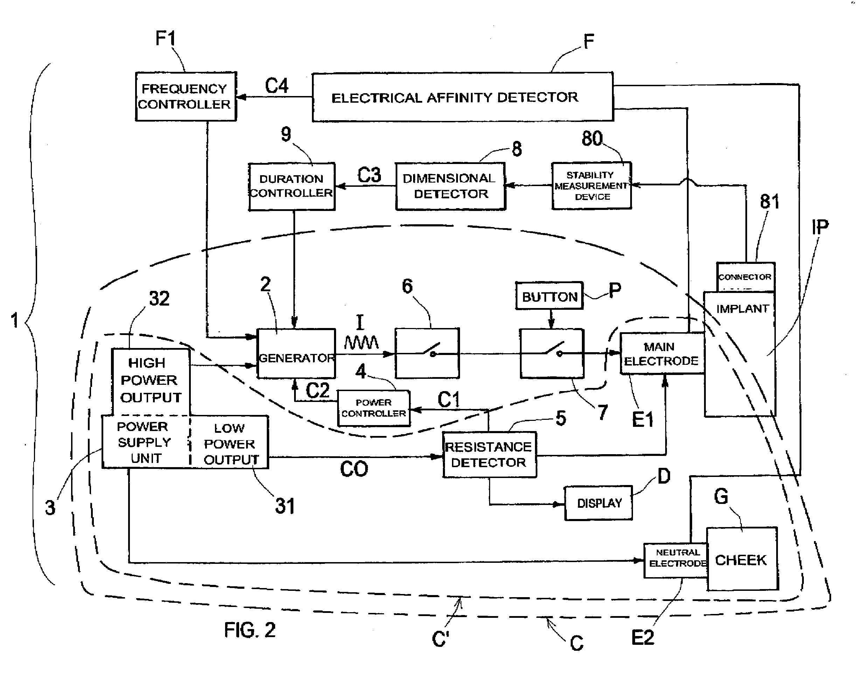

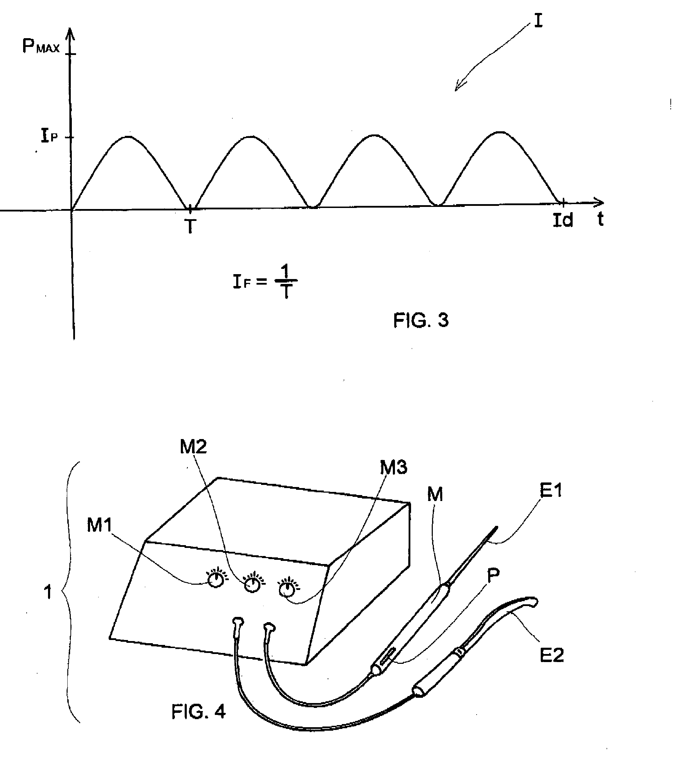

The device of the invention is described hereinafter, as illustrated in Figs. 2 and 4 and generally indicated with reference number (1).

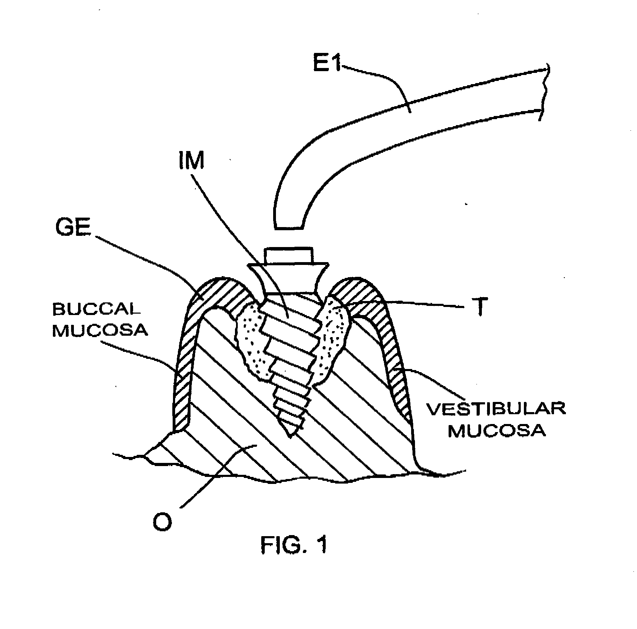

Referring now to Fig. 1, a dental implant (IM) is illustrated, which is implanted in a bone tissue (O) covered by the gum (GE). Fig. 1 shows the portions of the gum (GE) forming the buccal mucosa and vestibular mucosa. A damaged tissue (T) affected by perimplantitis is illustrated with dots in the portion of bone (O) and gum (GE) in the proximity of the dental implant (IM).

Referring to Fig. 2, the device (1) of the invention comprises:
- a pulse generator (2) adapted to generate a high frequency electrical pulse train (I);
- an electrical power supply (3) electrically connected to the pulse generator (2);
- a resistance detector (5) connected to the electrical power supply (3),
- a power controller (4) disposed between the resistance detector (5) and the pulse generator (2);
- a main electrode (E1) connected to the pulse generator (2) and resistance detector (5), and adapted to be positioned in contact with the implant (IM) (as shown in Fig. 1), and
- a neutral electrode (E2) connected to the electrical power supply (3) and adapted to be positioned in contact with the patient's cheek (G) in correspondence with the implant (IM).

In view of the above, when the main electrode (E1) is in contact with the implant (IM) and the neutral electrode (E2) is in contact with the patient's cheek (G):
- a first closed electrical circuit (C) is generated, comprising power supply (3), pulse generator (2), main electrode (E1), implant (IM), cheek (G) and neutral electrode (E2); and
- a second closed electrical circuit (C') is generated, comprising power supply (3), resistance detector (5), main electrode (E1), implant (IM), cheek (G) and neutral electrode (E2).

So, by means of the first closed electrical circuit (C), the pulse train (I) can be administered to the damaged tissue (T) through the surface of the implant (IM); whereas, by means of the second closed electrical circuit (C'), the resistance detector (5) can detect the electrical resistance of the damaged tissue (T) in the proximity of the implant (IM).

It is important that the neutral electrode (E2) is positioned on the cheek (G) in correspondence of the damaged tissue (T), otherwise the measurement of the electrical resistance may be unreliable.

The electrical power supply (3) advantageously comprises a low-power current outlet (31) that powers the resistance detector (5) and a high-power current outlet (32) that powers the pulse generator (2). In this way the resistance detector (5) receives a low voltage electrical current to detect the electrical resistance of the damaged tissue (T). Instead, the pulse generator (2) receives a high-voltage alternating current to generate the pulse train (I) to be sent to the implant (IM).

Referring to Fig. 3, the pulse train (I) is characterized by:
- frequency (If) from 200 to 700 KHz;
- duration (Id) from 80 to 160 ms; and
- power (Ip) from 40% to 100% of a predefined maximum power value P_{MAX}, able to develop a dissipated power of 200 WATT on the damaged tissue.

The main electrode (E1) is connected to a handpiece (M) (see Fig. 4) adapted to be held by the operator. Advantageously, the main electrode (E1) has a tip with 2 mm diameter in order to have a stable contact with the implant (IM) (see Fig. 1), carefully avoiding any direct contact of the electrode (E1) with the damaged tissue (T).

The implant (IM) works as an antenna that dissipates the energy associated with the passage of current in equipotential mode on the entire contact surface between the implant (IM) and the damaged tissue (T).

The device (1) also comprises a safety relay (6) and a switch (7) disposed between generator (2) and main electrode (E1).

The safety relay (6) is normally closed and opens when the detected resistance is such to develop a dissipated power higher than a predefined threshold value.

The switch (7) is normally open and is connected to a button (P) provided on the handpiece (M). When closing, the switch (7) activates the measurement of the resistance detector (5) and the transmission of the high-power pulse (I) by the pulse generator (2).

The operation of the device (1) of the invention is disclosed hereinafter.

Through the low power outlet (31) the power supply (3) transmits a low-voltage electrical signal (C0) to the main electrode (E1) disposed in contact with the implant (IM). The low-voltage electrical signal (C0) passes through the resistance detector (5). Consequently, the resistance detector (5) detects, instant by instant, the electrical resistance of the damaged tissue (T) according to the electrical current that passes through the closed electrical circuit (C) of the device (1). The electrical resistance value of the damaged tissue (T) is measured in KΩ.

Advantageously, the device (1) comprises a display (D) connected to the resistance detector (5). The display (D) shows the electrical resistance value detected by the resistance detector (5) instant by instant.

The resistance detector (5) transmits a control signal (C1) to the power controller (4) according to the electrical resistance detected. The power controller (4) receives the control signal (C1) of the resistance detector (5) and transmits a control signal (C2) to the generator (2) according to the electrical resistance detected by the resistance detector (5).

The power controller (4) can also be actuated manually with a knob (M3) (see Fig. 4).

The pulse generator (2) receives the control signal (C2) sent by the power controller (4) and generates an electrical pulse train (I) with power (Ip) proportional to the electrical resistance value detected by the electrical resistance detector (5).

The electrical pulse train (I) generated by the generator (2) is transmitted to the main electrode (E1) after manually actuating the button (P) provided on the handpiece (M) of the main electrode (E1). The button (P) is electrically connected to the switch (7) and controls said switch (7). When the button (P) is pressed by the operator, the switch (7) is closed, allowing for the electrical pulse train (I) to pass from the generator (2) to the main electrode (E1).

During treatment, the electrical power developed by the electrical pulse train (I) in the damaged tissue (T) must be maintained at a constant value of about 200 Watt. In fact, if the power dissipated in the damaged tissue (T) is higher than 200 Watt, the damaged tissue (T) will be damaged, whereas if the power dissipated in the damaged tissue is lower than 200 Watt, the electrical pulse train (I) delivered by the main electrode (E1) will be ineffective for the treatment of the damaged tissue (T).

However, it must be considered that the power dissipated by the damaged tissue (T) depends on the electrical resistance value of the damaged tissue. Therefore, the electrical power (Ip) of the pulse train (I) generated by the pulse generator varies according to the electrical resistance value detected by the resistance detector (5).

So, the power (Ip) of the pulse train (I) generated by the pulse generator (2) and delivered by the main electrode (E1) on the damaged tissue (T) varies case by case according to the electrical resistance value of the damaged tissue (T) in order to maintain the value of the electrical power dissipated on the damaged tissue (T) constant.

For illustrative purposes:
- for electrical resistance values of the damaged tissue (T) lower than 6 KΩ, the pulse generator (2) generates a pulse train with power (Ip) equal to 40% of the maximum power;
- for electrical resistance values of the damaged tissue (T) comprised between 6 KΩ and 12 KΩ, the pulse generator (2) generates a pulse train with power (Ip) equal to 60% of the maximum power;
- for electrical resistance values of the damaged tissue (T) comprised between 12 KΩ and 18 KΩ, the pulse generator (2) generates a pulse train with power (Ip) equal to 80% of the maximum power;
- for electrical resistance values of the damaged tissue (T) higher than 18 KΩ, the pulse generator (2) generates a pulse train with power (Ip) equal to 100% of the maximum power.

The relay (6) is normally closed and opens when it detects a power value (Ip) of the pulse train (I) generated by the generator (2) higher than 200 Watt. Consequently, said relay (6) prevents the passage of the electrical pulse train (I) from the generator (2) to the main electrode (E1), in contact with the implant (IM), when the power (Ip) of said electrical pulse train (I) is higher than the predefined value.

Optionally, the device (1) of the invention comprises:
- a dimensional detector (8) that calculates the extension of the damaged tissue (T); and
- a duration controller (9) electrically connected to the detector (8) and adapted to control the duration (Id) of the electrical pulse train (I) generated by the pulse generator (2).

The dimensional detector (8) transmits a control signal (C3) to the duration controller (9) in such manner that the duration (Id) of the electrical pulse train (I) generated by the generator (2) is controlled according to the extension of the damaged tissue (T).

Advantageously, the dimensional detector (8) receives data from one or more implant stability measurement devices (80) connected to the dimensional detector (8). According to the data received by the implant stability measurement devices (80), the dimensional detector (8) calculates an implant relative stability (IRS) value.

The duration (Id) of the electrical pulse train (I) generated by the generator (2) is controlled by the duration controller (9) according to the implant relative stability (IRS) values in inverse relation; the higher the IRS values, the lower the duration (Id) of the pulse train delivered. For illustrative, not limitative purpose, the duration (Id) of the pulse train can vary from 80 to 160 ms.

For merely illustrative, not limitative purposes, the implant stability measurement device (80) comprises an OSSTELL^{®} implant stability meter. The OSSTELL^{®} implant stability meter comprises a metal connector (81) adapted to be fixed to the osteointegrated implant (IP) to detect implant stability.

The OSSTELL^{®} implant stability meter is a device of known type used to measure the stability of an osteointegrated implant by means of a Resonance Frequency Analysis (RFA) technique. The RFA technique provides for fixing the connector (81) on the osteointegrated implant by means of a screw. The connector (81) is provided with upper part comprising a magnet that is stimulated by magnetic pulses emitted by an electronic device. The connector (81) mounted on the osteointegrated implant has two main resonance frequencies and vibrates in two perpendicular directions, of which a first direction in which the implant is more stable and a second direction in which the implant is less stable. According to the vibrations made by the connector (81) and detected by the electronic device the OSSTELL^{®} implant stability meter provides Implant Stability Quotient (ISQ) values comprised from 1 to 100. The higher the ISQ values, the more stable the implant, and therefore, the less severe the perimplantitis.

The OSSTELL^{®} implant stability meters sends the ISQ values to the dimensional detector (8) that converts them into implant relative stability values (IRS) sent to the duration controller (9) to control the duration (Id) of the electrical pulse train (I) generated by the generator (2).

Optionally, the device (1) also comprises:
- an electrical affinity detector (F) that measures the transmucous tissue electrical potential at the level of the damaged tissue (T), and
- a frequency controller (F1) electrically connected to the electrical affinity detector (F) that sets the frequency (I) generated by the generator (2) according to the electrical potential measured by the electrical affinity detector (F).

The electrical affinity detector (F) sends a control signal (C4) to the frequency controller (F1) according to the electrical affinity value measured. Therefore the frequency controller (F1) controls the frequency (If) of the electrical pulse train (I) in a frequency range from 200 to 700 khz.

Advantageously, the electrical affinity detector (F) comprises a direct current voltmeter (100) connected to the electrode (E1) disposed in contact with the implant (IM) and to the neutral electrode (E2) disposed on the cheek.

The measurement of the voltmeter (100) is made in mvolts and is read on the voltmeter display. Such a measurement is made by closing the switch (7) actuated by the button (P) on the handpiece (M).

So, the frequency (If) of the electrical pulse train (I) generated by the generator (2) is controlled by the frequency controller (F1) according to the electrical affinity values measured by the voltmeter (100).

When closing, the switch (7) activates the measurements of the resistance, dimensional and electrical affinity detectors (5, 8, F) in a sequence and the transmission of the high-power pulse (I) towards the implant (IM).

Referring to Fig. 4, the device (1) of the invention optionally comprises three knobs (M1, M2, M3) used to manually control the frequency (If), duration (Id) and power (Ip) of the electrical pulse train (I) generated by the generator (2).

## Claims

1. Device (1) for treatment of perimplantitis comprising:
- a pulse generator (2) adapted to generate a high frequency electrical pulse train (I);
- an electrical power supply (3) electrically connected to the pulse generator (2);
- a main electrode (E1) connected to the pulse generator (2) and adapted to be disposed in contact with an intraosseus dental implant (IM) affected by perimplantitis on the damaged tissue (T),
- a neutral electrode (E2) connected to the electrical power supply (3) and adapted to be positioned in close contact with the patient's cheek (G) in correspondence of the damaged tissue (T) affected by perimplantitis, in such manner to generate a closed electrical circuit (C) to deliver said pulse train (I) to the damaged tissue (T), **characterised in that** the device further comprises
- a resistance detector (5) disposed between the electrical power supply (3) and the main electrode (E1) in such manner to generate a second closed electrical circuit (C') to measure the electrical resistance of the damaged tissue (T),
- a power controller (4) disposed between the resistance detector (5) and the pulse generator (2) to control the power (Ip) of the pulse train (i) according to the electrical resistance of the damaged tissue,
wherein the pulse generator (2) is adapted to generate a pulse train (I) with:
- frequency (If) from 200 to 700 KHz;
- duration (Id) from 80 to 160 ms; and
- power (Ip) from 40% to 100% of a predefined maximum power value P_{MAX}, able to develop a dissipated power of 200 WATT on the damaged tissue.

2. The device (1) of claim 1, wherein the electrical power supply (3) comprises a low-power current outlet (31) that powers the resistance detector (5) and a high-power current outlet (32) that powers the pulse generator (2).

3. The device (1) of any one of the preceding claims, also comprising a safety relay (6) and a switch (7) disposed between the generator (2) and the main electrode (E1), wherein the safety relay (6) is normally closed and opens when a dissipated electrical power higher than the predefined threshold value is measured and the switch (7) is normally open and is connected to a button (P) disposed on a handpiece (M) connected to the main electrode (E1).

4. The device (1) of any one of the preceding claims, also comprising a display (D) connected to the resistance detector (5) to display the detected resistance.

5. The device (1) of any one of the preceding claims, also comprising:
- a dimensional detector (8) that calculates the extension of the damaged tissue (T); and
- a duration controller (9) electrically connected to the dimensional detector (8) and adapted to control the duration (Id) of the electrical pulse train (I) generated by the pulse generator (2) according to the extension of the damaged tissue.

6. The device (1) of claim 5, also comprising at least one implant stability measurement device (80) connected to the dimensional detector (8) to measure implant relevant stability (IRS) values, wherein said dimensional detector (8) transmits signals (C3) to the duration controller (9) that indicate the implant relative stability (IRS) measured by the stability measurement device (80).

7. The device (1) of claim 6, wherein said implant stability measurement device (80) comprises an OSSTELL implant stability meter provided with a metal connector (81) adapted to be fixed to the implant (IP) to detect the implant stability quotient (ISQ).

8. The device (1) of any one of the preceding claims, also comprising:
- an electrical affinity detector (F) that measures the transmucous electrical potential of the perimplantar damaged tissue (T), and
- a frequency controller (F1) electrically connected to the electrical affinity detector (F) that varies the frequency (I) of the electrical pulse train (I) generated by the pulse generator (2) according to the electrical affinity value detected by the electrical affinity detector (F).

9. The device (1) of claim 8, wherein said electrical affinity detector (F) comprises a voltmeter (100), the main electrode (E1) disposed in contact with the implant (IM) and the neutral electrode (E2) in contact with the cheek (G).

## Patentansprüche

1. Vorrichtung (1) für die Behandlung von Periimplantitis, umfassend:
- einen Impulserzeuger (2), der dazu geeignet ist, eine Folge von hochfrequenten Elektroimpulsen (i) zu erzeugen;
- ein elektrisches Netzteil (3), das elektrisch am Impulserzeuger (2) angeschlossen ist;
- eine Hauptelektrode (E1), die am Impulserzeuger (2) angeschlossen und dazu bestimmt ist, im Kontakt mit einem enossalen Zahnimplantat (IM), das von Periimplantitis an einem erkrankten Gewebe (T) betroffen ist, positioniert zu werden;
- eine Neutralelektrode (E2), die an dem elektrischen Netzteil (3) angeschlossen und dazu bestimmt ist, in engem Kontakt mit einer Wange (G) des Patienten im Bereich des an Periimplantitis erkrankten Gewebes (T) derart zu positioniert zu werden, dass ein geschlossener Stromkreis (C) gebildet wird, um die Impulsfolge in das erkrankte Gewebe (T) einzuleiten,
**dadurch gekennzeichnet, dass** die Vorrichtung ferner Folgendes umfasst:
- einen Widerstandsmesser (5), der zwischen dem elektrischen Netzteil (3) und der Hauptelektrode (E1) derart angeordnet wird, dass ein zweiter geschlossener Stromkreis (C') gebildet wird, um den Elektrowiderstand des erkrankten Gewebes (T) zu messen,
- einen Leistungsregler (4), der zwischen dem Widerstandsmesser (5) und dem Impulserzeuger (2) angeordnet wird, um die Leistung (Ip) der Impulsfolge (i) entsprechend dem Elektrowiderstand des erkrankten Gewebes zu regeln;
wobei der Impulserzeuger (2) vorgesehen ist, um eine Impulsfolge (I) mit folgenden Merkmalen zu erzeugen:
- eine Frequenz (If), die im Bereich zwischen 200 - 700 KHz liegt;
- eine Dauer (Id), die im Bereich zwischen 80 und 160 ms liegt; und
- eine Leistung (Ip), die zwischen 40% und 100% eines zuvor festgelegten Höchstleistungswerts P_{MAX} variieren kann und in der Lage ist, eine dissipierte Leistung von 200 WATT in dem erkrankten Gewebe zu erzeugen.

2. Vorrichtung (1) nach Anspruch 1, wobei das elektrische Netzteil (3) einen Ausgang für Niederfrequenzstrom (31) umfasst, der den Widerstandsmesser (5) versorgt, und einen Ausgang für Hochfrequenzstrom (32), der den Impulserzeuger (2) versorgt.

3. Vorrichtung (1) nach einem beliebigen der vorstehenden Ansprüche, umfassend ferner ein Sicherheitsrelais (6) und einen Schalter (7), welche zwischen dem Impulserzeuger (2) und der Hauptelektrode (E1) angeordnet sind, wobei das Sicherheitsrelais (6) normalerweise geschlossen ist und nur dann geöffnet wird, wenn eine dissipierte elektrische Leistung gemessen wird, die einen voreingestellten Schwellenwert überschreitet, und der Schalter (7) normalerweise geöffnet und mit einer Taste (P) verbunden ist, die an einem Handstück (M) angeordnet ist, das mit der Hauptelektrode (E1) verbunden ist.

4. Vorrichtung (1) nach einem beliebigen der vorstehenden Ansprüche, umfassend ferner ein Display (D), das mit dem Widerstandsmesser (5) verbunden ist, um den gemessenen Widerstand anzuzeigen.

5. Vorrichtung (1) nach einem beliebigen der vorstehenden Ansprüche, umfassend ferner:
- einen Größenmesser (8), der die Ausdehnung des erkrankten Gewebes (T) berechnet; und
- einen Dauerregler (9), der elektrisch mit dem Größenmesser (8) verbunden und dazu geeignet ist, die Dauer (Id) der Elektroimpulsfolge (I), die vom Impulserzeuger (2) erzeugt wird, entsprechend der Ausdehnung des erkrankten Gewebes zu regulieren.

6. Vorrichtung (1) nach Anspruch 5, umfassend ferner mindestens einen Implantatstabilitätsmesser (80), der mit dem Größenmesser (8) verbunden ist, um die Werte der relativen Implatatstabilität (IRS) zu messen, wobei der Größenmesser (8) an den Dauerregler (9) Signale (C3) sendet, die auf die relative Implatatstabilität (IRS) hinweisen, die vom Stabilitätsmesser (80) gemessen wird.

7. Vorrichtung (1) nach Anspruch 6, wobei der Implantatstabilitätsmesser (80) ein OSSTELL umfasst, das mit einem Metallverbinder (81) versehen und dazu bestimmt ist, am Implantat (IP) befestigt zu werden, um den Implantatstabilitätsquotienten (ISQ) zu ermitteln.

8. Vorrichtung (1) nach einem beliebigen der vorstehenden Ansprüche, umfassend ferner:
- einen Elektroaffinitätsmesser (F), der das transmuköse Elektropotential des periimplantaren Gewebes (T) misst, und
- einen Frequenzregler (F1), der elektrisch mit dem Elektroaffinitätsmesser (F) verbunden und dazu geeignet ist, die Frequenz (If) der Elektroimpulsfolge (I), die vom Impulserzeuger (2) erzeugt wird, entsprechend dem vom Elektroaffinitätsmesser (F) ermittelten Elektroaffinitätswert zu variieren.

9. Vorrichtung (1) nach Anspruch 8, wobei der Elektroaffinitätsmesser (F) ein Voltmeter (100) umfasst, die Hauptelektrode (E1) im Kontakt mit dem Implantat (IM) angeordnet ist und die Neutralelektrode (E2) sich im Kontakt mit der Wange (G) befindet.

## Revendications

1. Dispositif (1) pour le traitement de la péri-implantite comprenant :
- un générateur d'impulsions (2) pour générer un train d'impulsions électriques (i) à haute fréquence ;
- une alimentation électrique (3) connectée électriquement au générateur d'impulsions (2) ;
- une électrode principale (E1) branchée au générateur d'impulsions (2) et destinée à être positionnée en contact avec un implant dentaire intra-osseux (IM) affecté par une péri-implantite sur un tissu malade (T),
- une électrode neutre (E2) branchée à la source d'alimentation (3) et destinée à être placée en contact étroit avec la joue (G) du patient dont le tissu malade (T) est affecté par une péri-implantite, de manière à générer un circuit électrique fermé (C) pour alimenter ledit train d'impulsions (I) au tissu malade (T),
**caractérisé en ce que** le dispositif comprend en outre:
- une résistance de détection (5) disposée entre le bloc d'alimentation (3) et l'électrode principale (E1) de manière à générer un second circuit électrique fermé (C') de mesure de la résistance électrique du tissu malade (T),
- un régulateur de puissance (4) disposé entre la résistance de détection (5) et le générateur d'impulsions (2), pour régler la puissance (Ip) du train d'impulsions (i) en fonction de la résistance électrique du tissu malade,
où le générateur d'impulsions (2) est disposé de manière à générer un train d'impulsions (I) ayant :
- une fréquence (If) qui peut varier dans la plage 200 - 700 KHz ;
- une durée (Id) qui peut varier dans la plage de 80 à 160 ms ; et
- une puissance (Ip) qui peut varier de 40% à 100% d'une valeur de puissance maximale préfixée P_{MAX}, en mesure de développer une puissance dissipée sur le tissu malade de 200 WATT.

2. Dispositif (1) selon la revendication 1, où l'alimentation électrique (3) comprend une sortie de courant de puissance à basse tension (31) qui alimente la résistance de détection (5) et un courant électrique de sortie à haute tension (32) qui alimente le générateur d'impulsions (2).

3. Dispositif (1) selon l'une quelconque des revendications précédentes, comprenant en outre un relais de sécurité (6) et un commutateur (7) disposé entre le générateur (2) et l'électrode principale (E1), dans lequel le relais de sécurité (6) est normalement fermé et s'ouvre lorsqu'une puissance électrique dissipée qui dépasse une valeur de seuil prédéterminée est mesurée et le commutateur (7) est normalement ouvert et il est relié à un bouton (P) disposé sur un manche (M) relié à l'électrode principale (E1).

4. Dispositif (1) selon l'une quelconque des revendications précédentes, comprenant en outre un écran (D) branché au capteur de résistance (5) pour afficher la résistance détectée.

5. Dispositif (1) selon l'une quelconque des revendications précédentes, comprenant en outre
- un capteur dimensionnel (8) qui calcule l'extension du tissu malade (T) ; et
- un régulateur de durée (9) branché électriquement au capteur dimensionnel (8) et apte à régler la durée (Id) du train d'impulsions électriques (I) générées par le générateur d'impulsions (2), en fonction de l'extension du tissu malade.

6. Dispositif (1) selon la revendication 5, comprenant en outre au moins un mesureur de stabilité implantaire (80) branché au capteur dimensionnel (8) pour mesurer des valeurs de stabilité implantaire relative (IRS), où dit capteur dimensionnel (8) envoie au régulateur de durée (9) des signaux (C3) indicatifs de la stabilité implantaire relative (IRS) mesurée par le mesureur de stabilité (80).

7. Dispositif (1) selon la revendication 6, où ledit mesureur de stabilité implantaire (80) comprend un compteur OSSTELL muni d'un connecteur métallique (81) destiné à être fixé à l'implant (IP) pour relever le quotient de stabilité de l'Implant (ISQ).

8. Dispositif (1) selon l'une quelconque des revendications précédentes, comprenant en outre :
- un capteur d'affinité électrique (F), qui mesure le potentiel électrique trans-muqueux du tissu malade (T) affecté d'une péri-implantite, et
- un régulateur de fréquence (F1) branché électriquement au capteur d'affinité électrique (F) et apte à varier la fréquence (I) du train d'impulsions électriques (I) générées par le générateur d'impulsions (2) conformément à la valeur d'affinité électrique détectée par le capteur d'affinité électrique (F).

9. Dispositif (1) selon la revendication 8, où ledit capteur d'affinité électrique (F) comprend un voltmètre (100), l'électrode principale (E1) positionnée en contact avec l'implant (IM) et l'électrode neutre (E2) en contact avec la joue (G).
